# EUROPÄISCHE PATENTANMELDUNG

(11) **EP 3 295 911 A1**
(43) Veröffentlichungstag der Anmeldung: **21.03.2018**
(21) Anmeldenummer: 16189060.3
(22) Anmeldetag: 15.09.2016
(51) Int. Cl.: A61F 13/49, A61F 13/496, A61F 13/15

(54) **NEUARTIGE WIEDERVERWENDBARE SCHWIMMWINDEL**

(71) Anmelder: Lässig Holding GmbH, 64832 Babenhausen (DE)
(72) Erfinder: LÄSSIG, Claudia, 63739 Aschaffenburg (DE)
(74) Vertreter: Patentanwälte Gierlich & Pischitzis Partnerschaft mbB

(57) **Zusammenfassung**

Die Erfindung beschreibt eine wiederverwendbare Schwimmwindel (10), in Form eines Höschens, mit einem elastischen Abschluss (12) im Taillen und/oder Beinbereich, die Folgendes aufweist:
a) eine Außenschicht (14), wobei die Außenschicht aus einer elastischen Maschenware oder einer Webware ausgestaltet ist;
b) eine Innenschicht (16), wobei die Innenschicht aus einer Maschenware oder einer Webware aus natürlichen und/oder synthetischen Fasermaterialien ausgestaltet ist;
c) und wobei es sich bei der Innenschicht (16) um eine wasserdampfdurchlässige und wasserabsorbierende Innenschicht handelt.

## Beschreibung

### Gebiet der Erfindung

Die Erfindung betrifft eine wiederverwendbare Schwimmwindel in Form eines Höschens, mit einem elastischen Abschluss im Taillen- und/oder Beinbereich.

### Stand der Technik

In den letzten Jahrzehnten wurden wiederverwendbare Stoffwindeln, die bspw. aus Baumwolle bestehen, bei der Verwendung durch stark absorbierende Wegwerfwindeln ersetzt.

Dies ist teilweise dem Umstand geschuldet, dass Wegwerfwindeln in der Regel viel mehr Feuchtigkeit aufnehmen können als bspw. Stoffwindeln, leichter zu verwenden sind und bei Verschmutzung leicht entsorgt werden können.

Allerdings sind weder wiederverwendbare Stoffwindeln noch herkömmliche Wegwerfwindeln für Säuglinge oder Kleinkinder geeignet, um mit diesen zu schwimmen oder an Wasseraktivitäten teilzunehmen.

So sind sowohl stark absorbierende Wegwerfwindeln als auch wiederverwendbare Stoffwindeln bspw. zu sperrig und sie nehmen zu viel Wasser auf, um mit ihnen zu schwimmen oder an Wasseraktivitäten teilzunehmen. Das z.T. erheblich höhere Gewicht nach Aufnahme großer Mengen an Wasser stellt zusätzlich ein Sicherheitsrisiko dar, da dadurch ein Säugling oder Kleinkind während des Schwimmens behindert wird und schlimmstenfalls sogar ertrinken kann.

Zusätzlich zu potentiellen Sicherheitsrisiken sind sowohl absorbierende Wegwerfwindeln als auch Stoffwindeln häufig unbequem, wenn sie sich mit Wasser vollsaugen.

Viele Eltern wechseln daher häufig die Windeln von Kindern, wenn diese bspw. zum Schwimmen oder Baden gehen. Etabliert haben sich dabei Schwimmwindeln die nicht aus stark wasserabsorbierenden Materialien wie bspw. Baumwolle bestehen.

Diese Schwimmwindeln für Kinder müssen mehrere Materialeigenschaften aufweisen. Zum einen müssen Materialien verwendet werden, die wasserdicht in dem Sinne sind, dass nicht Wasser von außen (z.B. Bade-/Meerwasser) in die Windel eindringt oder sich die Schwimmwindel damit vollsaugt, wobei zu beachten ist, dass bei wiederverwendbaren Schwimmwindeln Bein- und Hüftabschlüsse sowie evtl. vorhandene Nähte nicht komplett dicht abschließen, sodass (geringe Mengen) an Wasser trotzdem eindringen können. Gleichzeitig müssen Materialien verwendet werden, die Wasser bzw. flüssige Körperflüssigkeiten wie z.B. Urin aus der Schwimmwindel nach außen transportieren. Die eingesetzten Materialien sollten demnach zum einen atmungsaktiv sein, zum anderen eine Wasserdampfdurchlässigkeit aufweisen. Höherpreisige Produkte sollten darüber hinaus noch ein Feuchtigkeitsmanagement (engl. Wicking) aufweisen, d.h. Materialien sollten zum Einsatz kommen, die nicht nur wasserdicht sind und dennoch Wasserdampf durchlassen (sogenannte Atmungsaktivität/Wasserdampfdurchlässigkeit), vielmehr sollten Materialien verwendet werden, die zwar selbst nicht wasserdicht sind, aber die auftretende Feuchtigkeit (z.B. Schweiß oder Urin) schnell und aktiv vom Körper wegtransportieren (Feuchtigkeitsmanagement/Wicking). Mit Wicking wird demnach ein Effekt bezeichnet, bei dem Nässe auf eine größtmögliche Materialfläche ausgebreitet wird, um so die Verdunstung zu beschleunigen.

Wasserdampf kann bspw. auf zwei Arten durch wasserdichte Membranen gelangen. Zum einen durch sogenannte mikroporöse Membranen. Mikroporös heißt, dass die Membran mikroskopisch kleine Löcher hat, die so groß sind, dass Wasserdampfmoleküle hinaus können, aber gleichzeitig so klein sind, dass die größeren Wassermoleküle nicht hinein können. Als mikroporöse Membranen sind bspw. GoreTex® und eVENT® bekannt. Beide basieren auf einer gereckten Polytetrafluorethylen (ePTFE) Membran. Gereckt heißt, dass die Poren auf die Größe gebracht werden, dass Wasserdampf hindurch kann.

Das Problem bei solchen mikroporösen Membranen ist, dass Körperöle, Schweiß, aber auch Cremes von der Membran angesaugt werden und Kanäle im ePTFE hinterlassen, durch die Feuchtigkeit in die Produkte gelangen. Um das zu verhindern, können die Membrane mit bspw. einer dünnen Polyurethan (PU) Schicht überzogen werden. Dadurch werden die Membranen dauerhaft dicht. Gleichzeitig schränkt eine solche PU-Schicht die Atmungsaktivität jedoch ein, weil sie Feuchtigkeit absorbiert und hält - weswegen entsprechende Produkte sich bei körperlicher Anstrengung innen nass anfühlen. Der Schweiß kondensiert an der PU-Schicht, wird dort absorbiert und wird langsam nach außen gedrückt.

Alternativ können sogenannte geschlossenzellige Membrane zum Einsatz kommen. Bei diesen lagert sich die Feuchtigkeit auf der Innenseite an. Die Membran quillt auf und die Wasserdampfmoleküle werden durch die Membran transportiert - eine Art Osmose-Prinzip. Dies funktioniert mit einer kleinen Zeitverzögerung. Geschlossenzellige Membranen gelten als deutlich robuster, weil sie keine Poren haben, die sich vergrößern (undicht) oder verstopfen (keine Atmung mehr) können. Die meisten geschlossenzellige Membran-Systeme sind aus Polyester.

Darüber hinaus gibt es noch wasserdichte Beschichtungen. Derer gibt es sehr viele und in erster Linie handelt es sich um (z.T. mikroporöse) Polyurethanbeschichtungen. In allen Fällen muss ein Partialdruckgefälle bestehen, um den Wasserdampfdurchgang zu ermöglichen.

Alle nicht-wasserdichten Stoffe lassen auch Wasserdampfmoleküle hindurch. Hierbei besteht jedoch das Problem, dass viele der Stoffe den Wasserdampf aufnehmen und speichern. Je nachdem, wie lange der Stoff dann nass ist beziehungsweise wie schnell er wieder trocken ist, entscheidet dies über die Funktionalität der Ware. Baumwolle zieht bspw. Feuchtigkeit schnell weg vom Körper, jedoch gibt sie diese nicht wieder ab. Baumwolle speichert bis zu 60% des Eigengewichts an Nässe. Das ist doppelt ungünstig: Zum einen kühlt die Nässe den Körper schnell aus und zum anderen kann die Membran, selbst wenn diese atmungsaktiv ist, nicht funktionieren, wenn die Schicht(en) darunter die Nässe speichern, sodass diese nicht heraus kann. Alternative Materialien sind die Poly-Stoffe, die bei Normalklima (20° C) und 65% Luftfeuchtigkeit eine Feuchtigkeitsaufnahme von etwa 7% des Eigengewichts (Acryl; (AC)), 0,5% für Polyester (PES), 4,5% für Polyamid (PA) und 0% für Polypropylene (PP) aufweisen. Weil auch zwischen 0% und 7% Feuchtigkeitsaufnahme ein spürbarer Funktionsunterschied besteht, gelten PES und PP als atmungsaktive Materialien.

Bestimmte Strickkonstruktionen mit unterschiedlichen Ebenen können auf mechanischem Wege Feuchtigkeit nach außen leiten. Doppellagige Materialien, also Stoffe, die aus zwei Komponenten mit unterschiedlichen Eigenschaften bestehen, leiten die Nässe aktiver nach außen weg. Teilweise spürt man dann noch die Nässe auf der Außenlage, während innen ein trockenes Gefühl besteht. Wichtig bei guten Wicking-Stoffen ist die schnelle Verteilung der Nässe auf möglichst großer Fläche. Dadurch kann die Feuchtigkeit besser verdampfen bzw. großflächiger an die nächste Schicht weitergegeben werden.

All diese Materialeigenschaften müssen idealerweise miteinander kombiniert werden um den Bedürfnissen der Eltern und der Kinder bei wiederverwendbaren Schwimmwindeln zu entsprechen.

Aus der US7678094B1 ist eine mehrlagige wiederverwendbare Schwimmwindel bekannt, deren einzelne Lagen bestimmte Materialeigenschaften wie Atmungsaktivität, Wasserdampfdurchlässigkeit etc. aufweisen. Nachteilig an der beschriebenen Schwimmwindel ist, dass bspw. die einzelnen Lagen miteinander durch Nahtführung zusammengehalten werden, was zur Reibung auf der sehr sensitiven Kinderhaut führen kann, wodurch Rötungen und Schwellungen hervorgerufen werden können. Weiterhin werden in der hautzugewandten Lage zwar Materialien mit einem "Wicking"-Effekt verwendet, nicht jedoch wie bei der vorliegenden Erfindung die "one-directional-wicking" Materialkombinationen, die den Feuchtigkeitskontakt mit der Haut auf ein Minimum reduzieren und somit ein angenehmes und hautfreundliches Empfinden hervorruft.

Aus der EP 15176716.7 ist eine mehrlagige wiederverwendbare Schwimmwindel bekannt, die aus mindestens 3 Lagen (Außen-, Zwischen- und Innenschicht) aufgebaut ist. Die Zwischenschicht ist im Wesentlichen im Schrittbereich der Schwimmwindel zwischen der Außen- und Innenschicht aufgebracht und soll gewährleisten, dass überschüssige Flüssigkeit/Wasser aufgenommen. Der Nachteil solcher mehrlagigen Schwimmwindeln mit Zwischenschichtanteil (sogenannte Patch-Konstruktionen) besteht u.a. darin, dass sich die gestaute Feuchtigkeit im relativ kleinflächigen Patch im unteren Schrittbereich konzentriert. Dadurch wird ein Herunterziehen der Schwimmwindel begünstigt, wenn die Zwischensicht/Patch mit Flüssigkeit gefüllt ist. Es resultiert eine Entfernung des Feuchtigkeitsspeichers von der Haut, was der Evaporation (Verdünstung) der Flüssigkeit auf der Haut entgegenwirkt.

### Aufgabe

Hiervon ausgehend liegt die Aufgabe der vorliegenden Erfindung darin, eine wiederverwendbare Schwimmwindel bereitzustellen, die einen hohen Tragekomfort aufweist und gleichzeitig Haut und Umwelt weitestgehend feuchtigkeitsfrei hält und somit das Einsatzgebiet derartiger Schwimmwindeln erweitert.

### Lösung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht. Die Erfindung umfasst auch alle sinnvollen und insbesondere alle erwähnten Kombinationen von unabhängigen und/oder abhängigen Ansprüchen.

Eine erfindungsgemäße wiederverwendbare Schwimmwindel ist in Form eines Höschens, mit einem elastischen Abschluss im Taillen und/oder Beinbereich, gestaltet. Weiterhin weist die Schwimmwindel auf:
a) eine Außenschicht, wobei die Außenschicht aus einer elastischen Maschenware oder einer Webware ausgestaltet ist;
b) eine Innenschicht, wobei die Innenschicht aus einer Maschenware oder einer Webware aus natürlichen und/oder synthetischen Fasermaterialien ausgestaltet ist;
c) und wobei es sich bei der Innenschicht um eine wasserdampfdurchlässige und wasserabsorbierende Innenschicht handelt.

Die Besonderheit dieser neu vorgestellten Windelkonstruktion besteht u.a. aus einer "Patch-losen" bzw. zwischenschichtfreien Innenkonstruktion, die es ermöglicht, dass innen, d.h. zur Haut hin, auftretende Feuchtigkeit auf einen um ein vielfaches größeren Speicher- und Evaporationsbereich ausgebreitet wird als herkömmliche Schwimmwindelkonstruktionen mit Patch. Dadurch wird ein angenehmeres Tragegefühl und mehr Bewegungsfreiheit erzeugt. Weiterhin wird als Folge der großflächigen Verteilung eine schnellere Evaporation bewirkt, die neben der größeren Flächenverteilung dadurch erzielt wird, dass sich nicht - wie bei herkömmlichen Patch-konstruktionen - der Feuchtigkeitsspeicher (wasserabsorbierende Innenschicht) im Schrittbereich befindet. Diese Distanz des Feuchtigkeitsreservoirs bei herkömmlichen Schwimmwindeln wirkt einer schnellen Evaporation dadurch entgegen, dass zur schnellen Verdunstung Körperwärme benötigt ist, welche die Feuchtigkeit von innen (Haut) nach außen (Umgebung) verdunsten lässt. Hieraus folgt, dass je näher die Feuchtigkeit an der Außenschicht liegt, desto mehr Evaporation erfolgt durch die entsprechend sich anstauende Körperwärme.

Bei der herkömmlichen Schwimmwindel entspricht jedoch das Evaporationsgebiet genau dem Bereich, der die größte Distanz der Feuchtigkeit von der Wärmequelle (Körper) aufweist.

Die erfindungsgemäße Schwimmwindel erzielt jedoch durch die Größe und Anordnung des Feuchtigkeitsspeichers über den gesamten Windelbereich eine um ein Vielfaches bessere Evaporation, da hier der Feuchtigkeitstransport zuerst in den Speicher hinein geschieht (one-directional wicking). Durch den Aufbau des Speichers in Faserform erfolgt der Feuchtigkeitstransport schnell und bevorzugt innerhalb der Speicherfasern. Die Feuchtigkeit wird dann weitflächig und in den gesamten Windelbereich hinein transportiert, d.h. bspw. auch in den Rücken und Bauchbereich, der durch die größere Körpernähe eine höhere Evaporationsrate erzielen kann (durch das one-directional wicking wird jedoch verhindert, dass die Haut in diesen Bereichen vermehrtem Feuchtigkeitskontakt ausgesetzt ist).

Im Vergleich zu sonstigen Schwimmwindelkonstruktionen unterscheidet sich die erfindungsgemäße Schwimmwindel dadurch, dass diese ein um ein Vielfaches größeren Feuchtigkeitsreservoir aufweist. Dieses Reservoir kann auftretende Feuchtigkeit solange aufnehmen, bis die Evaporation erfolgt ist. Andere Windelkonstruktionen führen üblicherweise zu einem vorzeitigen Austritt der Feuchtigkeit durch bspw. Nähte oder Beinabschlüsse, da die schnell austretende Feuchtigkeitsmenge in Ermangelung einer adäquaten Speicherkomponente keine entsprechende Feuchtigkeitsabsorption erfährt.

Der Ausdruck "elastischer Abschluss im Taillen- und/oder Beinbereich" umfasst sämtliche dem Fachmann bekannte Abschlüsse wie bspw. elastische Bünde, Stränge, Bänder, Filamente, Filamentwülste, Züge und dgl., die nahe bei einer Öffnung der wiederverwendbaren Schwimmwindel angeordnet sind, die ein Bein und/oder eine Taille eines Trägers aufnimmt. Ein Gummizug stellt bspw. einen bevorzugten elastischen Abschluss dar.

Dieser elastische Abschluss kann im Taillen- und/oder Beinbereich durch eine oder mehrere Nähte, bspw. zwei Nähte, an die wiederverwendbare Schwimmwindel angebracht werden.

Alternative Abschlüsse sind ebenfalls von der Erfindung umfasst. So können bspw. auch Knöpfe, Reiß- oder Klettverschlüsse und dgl. zum Einsatz kommen.

Die erfindungsgemäße Schwimmwindel weist eine Außenschicht auf, wobei die Außenschicht aus einer elastischen Maschenware oder einer Webware ausgestaltet ist.

Der Ausdruck "elastische Maschenware" umfasst sämtliche dem Fachmann bekannte textile Flächengebilde, bei denen eine mittels Faden gebildete Schleife in eine andere Schleife hineingeschlungen ist. Die auf diese Weise entstehenden Maschen können unter Verwendung eines oder mehrerer Fäden gebildet werden. Wirk- und Strickware gehören bspw. zu den Maschenwaren und sind erfindungsgemäß umfasst.

Der Vorteil von gewirkten Stoffen liegt in ihrer großen Elastizität. Für die erfindungsgemäße Schwimmwindel sind Gewirke daher hervorragend geeignet. Wolle und grobe Garne eignen sich zum Wirken ebenso wie sehr feine Garne. Beispiele für erfindungsgemäße elastische Maschenware sind bspw. elastische Maschenware aus Polyamid, Polypropylen, Polyester oder entsprechende Mischungen dieser Materialien, Elasthan (Block-Copolymer aus den Bestandteilen Polyurethan und Polyethylenglykol) oder anderen elastischen Fasern und deren Mischungen, Nylon-Gewirke und Gewirke, die unter Verwendung von elastischen Fasern hergestellt werden wie bspw. Pique, Fleece und Pannesamt.

Der Ausdruck "Webware" umfasst sämtliche dem Fachmann bekannte manuell oder maschinell gefertigten Erzeugnisse der Weberei wie bspw. Tuch, Samt, Velours, Plüsch, Frottee und sonstigen textilen Flächengebilden aus mindestens zwei rechtwinklig oder nahezu rechtwinklig verkreuzten Fadensystemen.

Gewebe sind bei hohen Fadendichten besonders widerstandsund strapazierfähig. Gesteigert wird dieser Effekt noch, wenn Zwirne anstelle von einfachen Garnen eingesetzt werden.

Gewebearten bzw. Webware, die erfindungsgemäß umfasst sind, können bspw. sein: Batist, Brokat, Chiffon, Chintz, Crepe (Krepp), Enoa, Damast, Denim, Drillich (Drell), Etamin (Siebtuch), Fil-a-fil, Flanell, Gabardine, Georgette, Jersey, Loden, Natte, Nessel, Pinpoint, Pique (Pikee), Popeline, Satin, Seersucker, Taft, Tuch, Tweed, Vollzwirn, Walkstoff.

In einer bevorzugten Ausführungsform zeichnet sich die wasserdampfdurchlässige und wasserabsorbierende Innenschicht dadurch aus, dass es sich hierbei um ein textiles Gewirke oder ein textiles Gebilde bzw. textiles Gewebe handelt. Vorzugsweise besteht dieses textile Gewirke oder dieses textile Gebilde bzw. textile Gewebe aus Gleichen oder verschiedenen Garnen und/oder Zwirnen.

Erfindungsgemäß werden unter Garne oder Zwirne alle Naturund synthetischen (chemischen) Fasern verstanden, die zu Garnen und Zwirnen verarbeitet werden.

Garn im Sinne von "Einfach-Garn" stellt hierbei ein einfädiges textiles Gebilde da, welches aus Spinnfasern oder Filamenten (Endlosfasern) besteht. Ein Spinnfasergarn besteht i.d.R. aus einer Anzahl von Spinnfasern, die durch Verdrehen (Spinnen) zusammengehalten werden. Ein Filamentgarn besteht i.d.R. aus einem oder mehreren Filament(en), die ohne oder mit Drehung hergestellt werden. Ein gefachtes Garn besteht aus zwei oder mehreren Garnen oder Zwirnen, die zusammen gespult, jedoch nicht miteinander verdreht sind.

Zwirn ist ein Sammelbegriff für alle linienförmigen, textilen Gebilde, die durch Zusammendrehen (Zwirnen) einfacher Garne und/oder Zwirne gleicher oder verschiedener Art entstanden sind. Einstufige Zwirne werden in einem Zwirnvorgang aus zwei oder mehr einfachen Garnen hergestellt. Mehrstufige Zwirne werden in einem oder mehreren zusätzlichen Zwirnvorgängen aus einstufigen und/oder mehrstufigen Zwirnen, gegebenenfalls auch unter Mitverwendung von einfachen Garnen, hergestellt. Durch das Zwirnen wird die Festigkeit des Fadens gesteigert und die Gleichmäßigkeit verbessert. Zwirne sind fester und widerstandsfähiger als Garne gleicher Feinheit. Durch entsprechende Abwandlung in der Verarbeitung kann man den Zwirnen Mustereffekte verleihen. Die bekanntesten Zwirne dieser Art sind Frotte-, Flammen-, Noppen-, Kräusel-, Schlingen- und Raupenzwirn. Bei Stoffbezeichnungen weist das Wort "Zwirn" darauf hin, dass es sich um ein besonders festes und dauerhaftes Material handelt, das aus gezwirnten Garnen besteht. Sämtliche Garne und/oder Zwirne sowie Mischungen davon sind von der Erfindung umfasst.

In einer weiteren bevorzugten Ausführungsform zeichnet sich die erfindungsgemäße wiederverwendbare Schwimmwindel dadurch aus, dass die als textile Gewirke, Gebilde und/oder Gewebe vorliegende Innenschicht als Einfachgestricke, Mehrfachgestricke und/oder Flächengebilde ausgestaltet ist. Hierbei können die textilen Gewirke, Gebilde und/oder Gewebe aus Gleichen oder unterschiedlichen Garnen und/oder Zwirnen bestehen. Dies kann sowohl innerhalb einer Materialebene als auch auf den verschiedenen Materialseiten (bspw. Ober- und Unterseite) erfolgen, abhängig davon, welche Materialeigenschaften gewünscht sind. Bevorzugt bestehen die Materialseiten des textilen Gewirkes oder des textilen Gebildes oder des textilen Geweben aus unterschiedlichen Garnen und/oder Zwirnen. So kann bspw. die der Haut zugewandte Materialseite aus anderen Garnen und/oder Zwirnen aufgebaut sein als die der Haut abgewandten (zur Umwelt zugewandten) Materialseite.

Erfindungsgemäß wird unter einer Mehrfachkonstruktion eine Konstruktion verstanden, die aus mehreren Schichten besteht, die gleichzeitig erstellt werden und die durch die Konstruktion selbst (z.B. Jacquard) oder aber durch zusätzliche Verarbeitungsschritte miteinander verbunden werden. Das Ergebnis von Mehrfachkonstruktionen sind in jedem Fall dauerhaft verbundene Flächen, die Hohlräume bieten können, die aber in sich eine geschlossene Lage bilden, da die Komponenten voneinander nicht getrennt werden können ohne die gesamte Konstruktion zu zerstören. Beispiele für derartige Mehrfachkonstruktionen die erfindungsgemäß umfasst werden ist z.B. ein Doppelgestrick, d.h. zwei übereinanderliegende Strickstühle erstellen jeweils eine Lage, die durch kreuzende Schussfäden verbunden sind, die ihrerseits fester Teil beider Lagen sind und zur Einzellagenkonstruktion beitragen. In die entstehenden Hohlräume können erfindungsgemäß Absorptionsfasern eingelegt werden, die durch ihr erhöhtes Absorptionsvermögen für die nötige Speicherkapazität als auch den dauerhaften Abzug der Feuchtigkeit aus der hautseitigen Materialschicht sorgen. Dem Fachmann sind weitere Einfachgestricke, Mehrfachgestricke und/oder Flächengebilde bekannt und werden von der Erfindung ebenfalls umfasst.

In einer weiteren bevorzugten Ausführungsform zeichnet sich die erfindungsgemäße Schwimmwindel dadurch aus, dass das textile Gewirke, textile Gebilde und/oder textile Gewebe Distanzstücke, Abstandshalter und/oder Faserkammern aufweist.

Vorzugsweise handelt es sich bei den Distanzstücken und/oder Abstandshalter um Abstandsgestricke, Abstandsgewirke und/oder Spacegarne.

Abstandsgestricke bestehen i.d.R. aus zwei durch einen Abstandsfaden miteinander verbundene Warenbahnen. Sie werden bspw. zur Herstellung von Sportbekleidungsstücken wie Trainings- oder Warmhalteanzügen sowie von Einlegewaren für den Sohlen- oder Seitenwandbereich von Schuhen verwendet

Zur Beeinflussung der Dehnbarkeit von Rechts/Links-Gestricken ist es insbesondere in Verbindung mit Plüschwaren bekannt, in ausgewählte Maschenreihen eines Grundgestricks wenigstens einen zusätzlichen Flottungs- bzw. Plattierfaden einzuarbeiten, der teilweise zu Maschen und teilweise zu Flottungen verarbeitet wird. Abstandsgestricke werden jedoch nicht auf Plüschmaschinen, sondern in der Regel auf Rundränderstrickmaschinen hergestellt, wobei die eine Warenbahn mit den Nadeln eines Nadelzylinders und die andere Warenbahn mit den Nadeln einer Rippscheibe gestrickt wird. Strickmaschinen dieser Art werden, sofern die Nadeln des Zylinders und der Rippscheibe auf Lücke stehen, auch als Feinrippmaschinen bezeichnet, zumindest wenn sie kleinere Nadelteilungen und große Durchmesser aufweisen. Ein Nachteil derartiger Strickmaschinen besteht darin, dass die beiden Warenbahnen jeweils mit allen Nadeln des einen oder anderen Nadelbetts hergestellt werden müssen, weshalb die Querelastizität und Querdehnung sowie andere Eigenschaften nur durch die Wahl des verwendeten Garns und nur in geringen Grenzen verändert werden können.

Unter einer Abstandskonstruktion (engl. Spaceyarn construction), wird im Wesentlichen eine Konstruktion verstanden, bei der mehrere flächenmäßig parallel liegende Flächen durch einen oder mehrere Legefäden (Spaceyarns) verbunden werden, und dabei entweder durch die Konstruktion selbst oder aber durch eine bedingte Steife des Legefadens Raum ("space") zwischen den Lagen generiert wird. Vorteile einer solchen Konstruktion bestehen u.a. darin, dass verschiedene Materialien mit unterschiedlichen Materialeigenschaften - bspw. in Bezug auf ihre Konstruktion als auch Komposition - miteinander verbunden werden können. So können bspw. Metallgarnkonstruktionen außen (kalt, hart, formgebend, wasserdicht etc.) und Velvetflor innen (soft, anpassende Form, warm, wasseraufsaugend etc.) miteinander verbunden werden.

Eine Spacekonstruktion ist eine Form der Mehrfachkonstruktion. Erfindungsgemäß ist vorgesehen, dass steifere Spacefäden eingesetzt werden, oder aber auf den Einsatz von Spacefäden verzichtet wird, jedoch die einzelnen Schichten durch einen Polfaden miteinander verbunden sind und der funktionsbedingende Hohlraum durch verschiedene Strickarten von Außen- und Innenlagen mit unterschiedlichen physikalischen Eigenschaften (Elastizität etc.) ausgefüllt wird. Darüber hinaus können auch feuchtigkeitsspeichernde Legefasern/Bauschfäden zwischen den Schichten liegen. Weitere erfindungsgemäß erfasste Konstruktionen umfassen Spacekonsruktionen oder eine 2- oder mehrkomponentige Schicht, die keine Legefasern/Bauschfäden aufweist. Generell werden alle dem Fachmann bekannten Konstruktionen, die Distanzstücke, Abstandshalter und/oder Faserkammern aufweisen, von der Erfindung umfasst.

Abstandsgewirke bzw. eine Mehrfachkonstruktion stellen doppelflächige Textilien dar, bei denen die kettengewirkten Warenflächen durch abstandshaltende Verbindungsfäden, sogenannte Polfäden, auf Distanz gehalten werden. Es handelt sich bei den Abstandsgewirken um Maschenwaren bzw. Gewirke, die um die dritte Dimension erweitert wurden.

Ein Abstandsgestrick oder Abstandsgewirk weist aufgrund seiner Dicke ein gutes Dämpfungs-, Feuchtigkeitstransport- sowie Feuchtigkeitsspeicherungsverhalten auf. Es ist bevorzugt, die Dicke des Abstandsgestricks oder Abstandsgewirks den zu erwartenden Belastungen beim Tragen der Schwimmwindel anzupassen. Beispielsweise könnte das Abstandsgestrick oder Abstandsgewirk im Bereich des Schrittes eine größere Dicke aufweisen als im Bereich des Rückens, um die Kraft- und Reibeeinwirkung im Schritt zielgerichtet zu verringern. Die Dicke des Abstandsgestricks kann im Bereich der kompletten Schwimmwindel variieren und könnte auch nur in einem vorgegebenen Bereich vorhanden sein.

Weiterhin bevorzugt weisen die Lagen des Abstandsgestricks oder Abstandsgewirks unterschiedliche Garne oder Zwirne auf. Hierdurch kann das Abstandsgestrick/-gewirk innerhalb der Schwimmwindel unterschiedliche Funktionen erfüllen. Beispielsweise kann die der Haut zugewandte Seite feuchtigkeitsabsorbierendes Garn und/oder Zwirn aufweisen, die der Haut abgewandten Seite gummiartiges Garn und/oder Zwirn aufweisen und das Garn und/oder der Zwirn zwischen diesen Seiten, d.h. das Abstandsgarn, stabiles Nylongarn sein. Weiterhin ist erfindungsgemäß umfasst, dass ein voluminöses und/oder hohles Garn oder Zwirn, welches Stoßkräfte aufnehmen kann, sowie ein feuchtigkeitsabsorbierendes Garn und/oder Zwirn, verwendet werden.

In diesem Zusammenhang ist es wichtig darauf hinzuweisen, dass die erfindungsgemäße Schwimmwindel bzw. die erfindungsgemäße Innenschicht die nachfolgenden Funktionen aufweist.
a. Sie zeichnet sich durch eine spezielle Feuchtigkeitsmanagement-Funktion (one-directional wicking) aus. Diese Funktion bewirkt, dass die auf der Windelinnenseite auftreffende Flüssigkeit sofort auf eine weitere Materialkomponente der Innenschicht transportiert wird, die als Feuchtigkeitsspeicher dient, und somit die Feuchtigkeit auf der dem Körper zugewandten Innenseite abzieht.
b. Sie zeichnet sich durch einen Feuchtigkeitsspeicher (absorbing component) aus. Die Feuchtigkeit wird hierbei bis zur Evaporation auf der Windelaußenseite zwischengespeichert. Konstruktiv kann bei der erfindungsgemäßen wiederverwendbaren Schwimmwindel das erhöhte Wasseraufnahmevermögen u.a. dadurch erzielt werden, dass Speicherfasern sowie spezifische Garnkonstruktionen wie bspw. Bauschgarne zum Einsatz kommen, die einen bevorzugten Feuchtigkeitstransport entlang und innerhalb von Speicherfaserkammern bevorzugen gegenüber einer Abgabe an die Außenschichten. Die Ausrichtung dieser Absorptionskammern innerhalb der erfindungsgemäßen Schwimmwindel kann entsprechend ausgerichtet sein, um einen Feuchtigkeitstransport innerhalb und entlang der Faserkanäle zu steuern, sodass die Feuchtigkeit beim Tragen der Schwimmwindel in die verdunstungsfreudigste Bereiche gelangen kann.
c. Die körperentfernte/hautentfernte Materialseite der Innenschicht hat eine quick-dry Ausrüstung, die für die schnelle Ausdunstung der Feuchtigkeit durch den Oberstoff bzw. Außenschicht verantwortlich ist. Dadurch kann sich die gespeicherte Feuchtigkeit sofort flächenhaft zum Verdunsten ausbreiten (wicking/quick-dry Ausrüstung).

Die beschriebenen Funktionen können aufgrund einer spezifischen Konstruktion in Maschen- oder Webtechnik erzielt werden, die aus einem Komplex von verschiedenen Faserkomponenten mit unterschiedlichem hydrophilen/hydrophoben Eigenschaften besteht. Geeignet hierfür ist bspw. eine Polyester-Maschenware.

Darüber hinaus vervollständigen Oberflächenbehandlungen des fertigen Materials den komplexen Funktionsprozess. Die erfindungsgemäße eingesetzte Kombination aus herkömmlicher quick-dry Ausrüstung mit dem erfindungseigenen "one-directional-wicking" finish macht so die wiederverwendbare Schwimmwindel zu einem einzigartigen Produkt, da der Feuchtigkeitskontakt mit der Haut auf ein Minimum reduziert wird. Im Vergleich zu handelsüblichen Schwimmwindeln, in denen durch die einfache "Wicking" Funktion genau der gegenteilige Zustand entsteht: Die Feuchtigkeit wird durch "Wicking"-Ausrüstung auf ein größtmögliches Ausmaß ausgebreitet, d.h. maximaler Körperkontakt zur Feuchtigkeit. Dieser Feuchtigkeitskontakt ist nicht nur unangenehm und hautunfreundlich, er wird noch verstärkt durch die wasserdichte Ausrüstung des nebenliegenden Außenmaterials handelsüblicher Schwimmwindeln, die den Feuchtigkeitsabtransport durch Verdunstung erheblich behindert bzw. unmöglich macht.

Alternativ ist die Innenschicht nicht einstückig als Abstandsgestrick oder Abstandsgewirk gefertigt, sondern separat gefertigt (z. B. gestrickt) und anschließend zusammengefügt (z.B. vernäht).

Es ist bevorzugt, dass Zwischenräume im Abstandsgestrick oder Abstandsgewirk mit dämpfenden Materialien gefüllt werden, um zusätzliche Funktionen zu erzielen. Beispielsweise könnten die Zwischenräume mit Fasern, Garnen (Spacegarnen), Kammern (Faserkammern) und/oder zusätzlichen Fasern aufgefüllt werden.

Weiter bevorzugt ist, dass diese Materialien austauschbar sind, damit der Benutzer die Eigenschaften seinen Bedürfnissen anpassen kann. Beispielsweise könnte die Maschenware im Schrittbereich so gestrickt sein, dass sie Öffnungen, Taschen und/oder Tunnel aufweist, welche die Materialien austauschbar aufnehmen können.

In einer weiteren bevorzugten Ausführungsform zeichnet sich die wiederverwendbare Schwimmwindel dadurch aus, dass die textilen Gewirke, textilen Gebilde und/oder textilen Gewebe im Wesentlichen ohne Nahtführung miteinander verbunden sind. Dies kann bspw. mittels Welding (Druckschweißen), vorzugsweise Ultrasonic Welding (Ultraschallschweißen), Verkleben und/oder Verschmelzen erfolgen und so gewährleisten, dass eine dauerhafte Verbindung erzielt wird.

Das sogenannte Ultrasonic Welding oder auch Ultraschallschweißen ist eine industrielle Technik, bei der hochfrequenter Ultraschall lokal angewendet werden, um Werkstücke unter Druck zusammenzuhalten und so einen Festkörper zu schaffen (Schweißung). Es wird allgemein für die verwendeten Kunststoffe, und insbesondere für unterschiedliche Verbindungsmaterialien, eingesetzt.

In einer weiteren besonders bevorzugten Ausführungsform zeichnet sich die wiederverwendbare Schwimmwindel dadurch aus, das die wasserdampfdurchlässige und wasserabsorbierende Innenschicht aus einer Maschenware oder einer Webware aus synthetischen Fasermaterialien besteht, vorzugsweise eine Polyester Innenschicht, insbesondere eine Polycarbonat (PC), Polyethylenterephthalat (PET) und/oder Polyesterharz Innenschicht.

Erfindungsgemäß werden unter Polyester alle Polymere mit Esterfunktionen -[-CO-O-]- in ihrer Hauptkette umfasst. Hierunter fallen bspw. die synthetische Polymere (Kunststoffe), zu denen die viel verwendeten Polycarbonate (PC), Polyethylenterephthalat (PET) und das duroplastische ungesättigte Polyesterharz (UP) gehören. Weitere Fasern aus synthetischen Polymeren die erfindungsgemäß umfasst sind, sind bspw. Polyamide (PA), Polyimide (PI), Polyamidimide (PAI), Polyphenylensulfide (PPS), Aramide, Polyacrylnitrile (PAN), Polytetrafluorethylene (PTFE), Polyethylene (PE), Polypropylene (PP), Polyvinylchloride (PVC bzw. bei Fasern CLF) und Polyurethane (EL).

Besonders bevorzugt ist die wasserdampfdurchlässige und wasserabsorbierende Innenschicht aus Materialien aufgebaut, die "one-directional wicking" Eigenschaften aufweisen. Erfindungsgemäß wird hierunter eine Weiterentwicklung des herkömmlichen "wicking" verstanden, bei dem der Feuchtigkeitsgehalt des Materials nicht nur bezüglich der Größe, sondern auch der Position geregelt wird. Das heißt, dass die auf der Stoffoberseite auftreffende Feuchtigkeit zuerst und schnell auf die Stoffunterseite gezogen wird, von wo dann die Wickingfunktion (=größtmögliches Verteilen der Feuchtigkeit) stattfindet. Daraus folgt, dass auf der Materialoberseite (bei der erfindungsgemäßen Schwimmwindel handelt es sich hierbei um die dem Körper zugewandte Materialseite) nur kleine Mengen an Restfeuchtigkeit verbleiben, während die überwiegende Menge an Feuchtigkeit auf die dem-Körper-abgewandte Seite abgezogen wird, um dort zu verdunsten. Im Vergleich dazu wird beim herkömmlichen Wicking der Feuchtigkeitsgehalt auf der Stoffober- und -unterseite gleichmäßig und über eine große Fläche verteilt, was zu einem entsprechend "größtmöglichen und flächigen" Kontakt der (sensiblen) (Klein-)Kinderhaut mit der angestauten Feuchtigkeit führt und zu Hautirritationen, Rötungen, Reibungen, vermindertem Tragekomfort etc. führen kann. Materialien die entsprechende "onedirectional-wicking" Eigenschaften aufweisen und demgemäß als wasserdampfdurchlässige Innenschicht verwendet werden können sind bspw. die beschriebenen synthetischen Fasermaterialien, insbesondere die Polyester Fasermaterialien. Dem Fachmann sind weitere Kunstfaser/-mischungen bekannt, die erfindungsgemäß zum Einsatz kommen können.

In einer weiteren bevorzugten Ausführungsform handelt es sich bei den wasserabsorbierenden Komponenten der Innenschicht um eine Frottee- und/oder Fleece-Innenschichtkomponente. Frottee bietet den Vorteil, dass es besondere Saugfähigkeit aufweist und gleichzeitig einen angenehmen Griff hat. Bevorzugt ist die wasserabsorbierend Innenschichtkomponenten im Wesentlichen über die gesamte Schwimmwindel verteilt, d.h. über die gesamte Innenschicht aufgebracht.

Weiterhin besteht die Außenschicht in einer bevorzugten Ausführungsform aus wasserdichten Materialien oder Mischungen aus wasserdichten und wasserdurchlässigen Materialien.

In einer weiteren bevorzugten Ausführungsform zeichnet sich die wiederverwendbare Schwimmwindel dadurch aus, dass die Außenschicht eine Beschichtung und/oder eine Membran, vorzugsweise eine mikroporöse, hydrophobe und/oder hydrophile Membran, aufweist. Diese Beschichtung und/oder Membran kann mit weiteren Schichten flächig verbunden sein. Diese weiteren Schichten können ebenfalls Textilien, Folien aus Kunststoff oder Metall, Schaumstoff oder andere sein. Die Verbindung erfolgt stoffschlüssig beispielsweise durch Verkleben oder Verschmelzen auf speziellen Textilmaschinen (Lamination). Die Beschichtung und/oder die Membran soll insbesondere die Wasserdichtigkeit der wiederverwertbaren Schwimmwindel gewährleisten bzw. erhöhen.

Durch die Lamination entsteht ein Material, das die Eigenschaften seiner Ausgangsmaterialien kombiniert: So liefert bspw. ein kräftiges Gewebe Reiß- und Abriebfestigkeit, eine Kunststofffolie Wasser- und Winddichtigkeit, und die Verbindung mit einer Metallfolie ergibt ein lichtundurchlässiges Material, das unter Umständen auch noch Wärmestrahlung reflektiert. Die Außenschicht kann bspw. mit einer sehr dünnen Membranfolie laminiert sein, die winddicht, atmungsaktiv und/oder wasserdicht ist.

Bei den Beschichtungen können sowohl Faserbeschichtungen als auch Flächenbeschichtungen zum Einsatz kommen. Wird z.B. Teflon als Beschichtung eingesetzt (und nicht als Membran), werden die Fasern mit dem Kunststoff ummantelt.

Bei einer Flächenbeschichtung, welche meist auf ein Trägermaterial aufgedampft oder aufgestrichen wird, können einfache oder mikroporöse Flächenbeschichtungen erfindungsgemäß eingesetzt werden. Bei der einfachen Variante (wasserdicht aber nicht atmungsaktiv) wird die Gewebefläche geschlossen beschichtet, es kann kein Wasser mehr eindringen, aber es geht auch keine Feuchtigkeit heraus.

Bei den mikroporösen Membranen (wasserdicht und atmungsaktiv) ist der Beschichtungsfilm von winzigen Poren durchsetzt, die ein Eindringen von Feuchtigkeit verhindern, aber ein Austreten von Wasserdampf ermöglichen. Bei diesen Beschichtungen bleibt die Atmungsaktivität erhalten. Gleiche Charakteristika weisen die hydrophilen und hydrophoben Membranen auf.

Für die Verarbeitung von Membranen auf Textilien gibt es verschiedene Möglichkeiten: Beim Oberstofflaminat wird die Membran direkt mit dem Oberstoff als Zweilagen-Laminat verbunden. Beim Insertlaminat wird die Membran auf ein leichtes Trägermaterial laminiert und zwischen Oberstoff und Futter verarbeitet. Beim Futterlaminat wird der Futterstoff auf der linken Seite mit der Membran verbunden. Beim Dreischichtlaminat wird die Membran nicht nur mit dem Oberstoff, sondern auch mit dem Futterstoff verbunden.

Von der Erfindung umfasst sind sämtliche beschriebene sowie dem Fachmann bekannte Verarbeitungsverfahren zum Aufbringen einer Beschichtung und/oder einer Membran auf Textilien.

Eine erfindungsgemäß bevorzugte Beschichtung ist bspw. eine Kunststoff- und/oder Kunstharz Beschichtung, wie bspw. eine Polyurethan- (PU) oder Polyvinylchlorid (PVC) Beschichtung. Die PU-Beschichtungen haben den Vorteil, dass sie im Vergleich zu PVC-Beschichtungen leichter sind, auch bei niedrigen Außentemperaturen elastisch bleiben und umweltverträglicher sind.

In einer weiteren bevorzugten Ausführungsform handelt es sich bei der Membran vorzugsweise um eine mikroporöse Membran, insbesondere um eine synthetische und/oder halbsynthetische Polymermembran, vorzugsweise eine Polytetrafluoroethylen (PTFE) Membran, insbesondere eine gereckte Polytetrafluoroethylen (PTFE) Membran.

Die erfindungsgemäße wiederverwendbare Schwimmwindel zeichnet sich u.a. auch dadurch aus, dass die Beschichtung und/oder (mikroporöse) Membran im Wesentlichen ohne Nahtführung auf die Außenschicht aufgebracht ist. Ein Verfahren, um solche nahtlosen Verbindungen herzustellen, stellt bspw. das sogenannte Welding (Druckschweißen) dar. Alternative Verfahren sind bspw. das schon beschriebene Verkleben/Verschmelzen auf speziellen Textilmaschinen. Eine besonders bevorzugte wiederverwendbare Schwimmwindel zeichnet sich daher dadurch aus, dass die Beschichtung und/oder (mikroporöse) Membran mittels Welding (Druckschweißen) dauerhaft auf die Außenschicht aufgebracht ist. Generell ist darauf zu achten, dass nach Möglichkeit eine nahtfreie Verbindung zwischen Außenschicht, Beschichtung und/oder (mikroporöse) Membran vorliegt, da eine Naht ein perforieren erfordert und somit die Wasserdichtigkeit eingeschränkt wird.

Ergänzend hierzu kann jede Außenschicht noch einen Sonnenschutzfaktor, genauer einen UV-Schutzfaktor (USF) oder Ultraviolet Protection Factor (UPF) aufweisen. Bevorzugt weist die Außenschicht einen UPF > 15 auf, besonders bevorzugt von > UPF 40. Der Sonnenschutz wird auf die Außenschicht mittels Ein- oder Aufbringen von UV- absorbierenden oder -reflektierenden Agenzien, durch Verwendung besonders dicht gewebter Stoffe oder durch bewusst eingesetzter Farbauswahl, erwirkt.

In einer weiteren bevorzugten Ausführungsform ist die wiederverwendbare Schwimmwindel dadurch gekennzeichnet, dass die Beschichtung und/oder mikroporöse Membran im Wesentlichen ohne Nahtführung auf die Außenschicht aufgebracht ist. Bevorzugte Verfahren zum Nahtfreien Aufbringen der Beschichtung und/oder einer mikroporösen Membran sind bspw. Welding (Druckschweißen), Verkleben und/oder Verschmelzen, sodass eine dauerhafte Verbindung mit der Außenschicht erzielt wird.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:
Fig. 1 Eine Darstellung einer Ausführungsform der wiederverwendbaren Schwimmwindel;
Fig. 2 Eine Gegenüberstellung von Materialien mit normaler "Wicking" und "one-directional-wicking" Eigenschaften;
Fig. 3 Darstellung einer geweldeten Froteeschicht;
Fig. 4 Darstellung verschiedener Ausführungsformen der Innenschicht der wiederverwendbaren Schwimmwindel;
Fig. 5 Schematische Darstellung des Schichtaufbaus einer Ausführungsform der wiederverwendbaren Schwimmwindel.

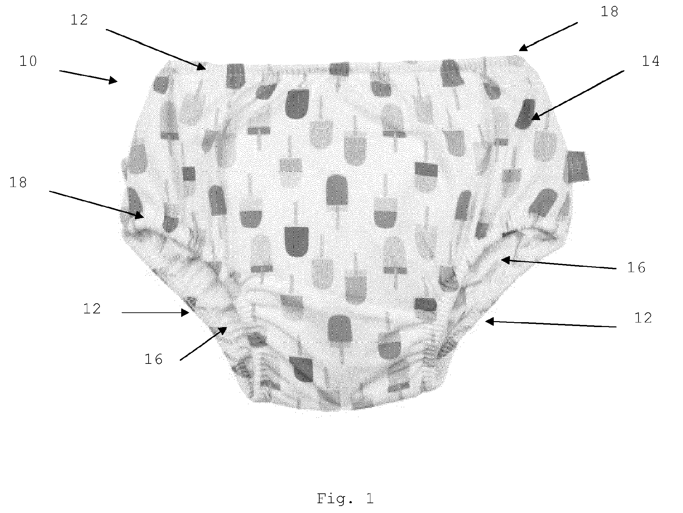

Fig. 1 zeigt eine Ausführungsform der wiederverwendbaren Schwimmwindel (10) in Form eines Höschens, mit einem elastischen Abschluss, vorliegend eines Gummizuges, im Taillen und/ Beinbereich (12) sowie eine Außenschicht (14) und eine Innenschicht (16). Ferner zeigt Fig. 1, dass der elastische Abschluss mittels einer Naht (18) an die wiederverwendbare Schwimmwindel (10) befestigt ist.

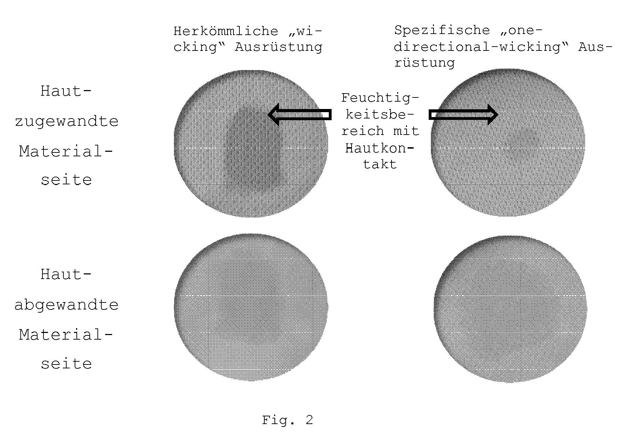

Fig. 2 zeigt eine Gegenüberstellung von Materialien mit normaler "Wicking" und "one-directional-wicking" Eigenschaften. Deutlich ist zu erkennen, wie bei den "one-directional-wicking" Materialien die auf der Stoffoberseite auftreffende Feuchtigkeit zuerst und schnell auf die Stoffunterseite gezogen wird, von wo dann die Wickingfunktion (=größtmögliches Verteilen der Feuchtigkeit) stattfindet. Daraus folgt, dass auf der Materialoberseite (bei der erfindungsgemäßen Schwimmwindel handelt es sich hierbei um die dem Körper zugewandte Materialseite) nur kleine Mengen an Restfeuchtigkeit verbleiben, während die überwiegende Menge an Feuchtigkeit auf die dem-Körper-abgewandte Seite abgezogen wird, um dort zu verdunsten. Im Vergleich dazu wird beim herkömmlichen Wicking der Feuchtigkeitsgehalt auf der Stoffober- und -unterseite gleichmäßig und über eine große Fläche verteilt, was zu einem entsprechend "größtmöglichen und flächigen" Kontakt der Haut mit der angestauten Feuchtigkeit führt.

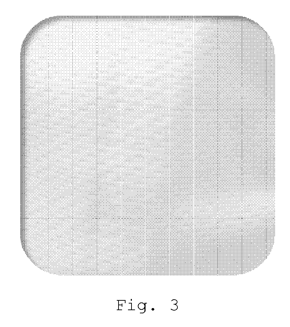

Fig. 3 zeigt einen zusätzlichen Feuchtigkeitsspeicher in Form eines geweldeten Frotteematerials. Dieses kann erfindungsgemäß als Innenschicht(komponenten) genutzt werden, wobei - wie weiter oben dargelegt - die Verbindung der Innenschicht mit der Außenschicht nahtlos, vorliegend mittels "Welding" (Druckschweißens) erfolgen kann.

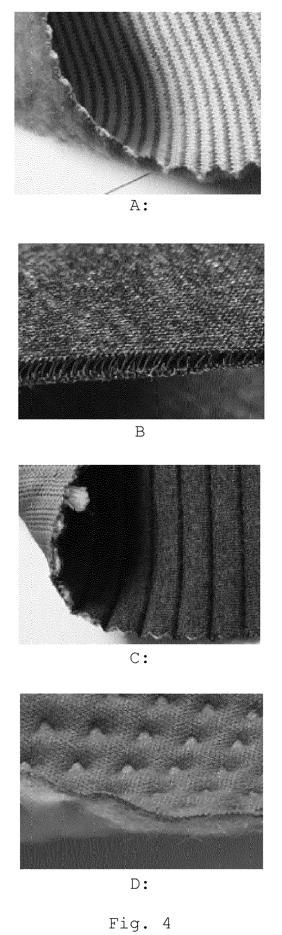

Fig. 4 zeigt den Aufbau verschiedener erfindungsgemäßer Innenschichten: 4A: Innenschicht als Einfachgestricke mit verschiedenen Garnkomponenten an beiden Materialseiten ausgestaltet; 4B: Innenschicht als Mehrfachgestricke mit Spacegarnen als Abstandshalter ausgestaltet; 4C: Innenschicht als Mehrfachgestricke mit Faserkammern ausgestalte; 4D: Innenschicht als komponentiges Flächengebilde, in der zwei Funktionskomponenten dauerhaft mittels flächendeckendem Ultrasonic Welding miteinander verbunden sind, ausgestaltet.

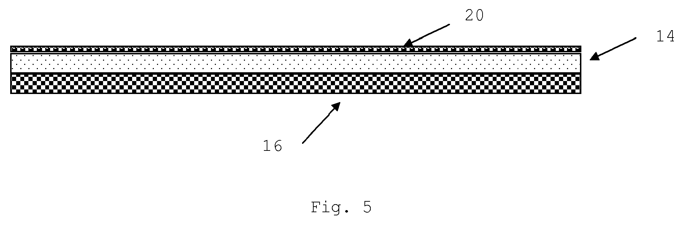

Fig. 5 zeigt eine schematische Darstellung des Schichtaufbaus der wiederverwendbaren Schwimmwindel. Gezeigt ist die Außenschicht (14), die Innenschicht (16), sowie eine Beschichtung (20), die auf die Außenschicht (14) aufgebracht ist und die Wasserdichte gewährleisten soll. Ferner ist dargelegt, dass die Innen- (16) und Außenschicht (14) ohne Nahtführung aufeinander befestigt sind. Dies kann bspw. mittels Welding (Druckschweißen), Verkleben oder Verschmelzen auf speziellen Textilienmaschinen erfolgen. Die Beschichtung (20) ist ebenfalls ohne Nahtführung auf die Außenschicht (14) aufgebracht worden. Die Verbindung erfolgt dabei stoffschlüssig beispielsweise durch Verkleben oder Verschmelzen auf speziellen Textilmaschinen (Lamination) oder mittels Welding (Druckschweißen).

Es sind zahlreiche Abwandlungen und Weiterbildungen der beschriebenen Ausführungsbeispiele verwirklichbar.

### Bezugszeichen

- 10: wiederverwendbare Schwimmwindel
- 12: elastischer Abschluss
- 14: Außenschicht
- 16: Innenschicht
- 18: Naht
- 20: Beschichtung

### zitierte Literatur

### zitierte Patentliteratur

US7678094B1
EP 15176716.7

## Patentansprüche

1. Wiederverwendbare Schwimmwindel, in Form eines Höschens, mit einem elastischen Abschluss im Taillen und/oder Beinbereich, **umfassend:**
a) eine Außenschicht, wobei die Außenschicht aus einer elastischen Maschenware oder einer Webware ausgestaltet ist;
b) eine Innenschicht, wobei die Innenschicht aus einer Maschenware oder einer Webware aus natürlichen und/oder synthetischen Fasermaterialien ausgestaltet ist;
c) und wobei es sich bei der Innenschicht um eine wasserdampfdurchlässige und wasserabsorbierende Innenschicht handelt.

2. Wiederverwendbare Schwimmwindel nach Anspruch 1, **dadurch gekennzeichnet, dass** es sich bei der wasserdampfdurchlässigen und wasserabsorbierenden Innenschicht um ein textiles Gewirke oder ein textiles Gebilde handelt.

3. Wiederverwendbare Schwimmwindel nach Anspruch 2, **dadurch gekennzeichnet, dass** das textile Gewirke oder das textile Gebilde aus Gleichen oder verschiedenen Garnen und/oder Zwirnen besteht.

4. Wiederverwendbare Schwimmwindel nach Anspruch 2 oder 3, **dadurch gekennzeichnet, dass** das textile Gewirke oder das textile Gebilde als Einfachgestricke, Mehrfachgestricke und/oder Flächengebilde ausgestaltet ist.

5. Wiederverwendbare Schwimmwindel nach einem der vorhergehenden Ansprüche, **dadurch gekennzeichnet, dass** die Materialseiten des textilen Gewirkes oder des textilen Gebildes aus unterschiedlichen Garnen und/oder Zwirnen bestehen.

6. Wiederverwendbare Schwimmwindel nach einem der vorhergehenden Ansprüche , **dadurch gekennzeichnet, dass** das textile Gewirke oder das textile Gebilde Distanzstücke, Abstandshalter und/oder Faserkammern aufweist.

7. Wiederverwendbare Schwimmwindel nach Anspruch 6, **dadurch gekennzeichnet, dass** es sich bei den Distanzstücken und/oder Abstandshalter um Abstandsgestricke, Abstandsgewirke und/oder Spacegarne handelt.

8. Wiederverwendbare Schwimmwindel nach einem der vorhergehenden Ansprüche, **dadurch gekennzeichnet, dass** textile Gewirke oder textile Gebilde im Wesentlichen ohne Nahtführung miteinander verbunden sind.

9. Wiederverwendbare Schwimmwindel nach Anspruch 8, **dadurch gekennzeichnet, dass** das textile Gewirke oder textile Gebilde mittels Welding (Druckschweißen), vorzugsweise Ultrasonic Welding (Ultraschallschweißen), Verkleben und/oder Verschmelzen dauerhaft miteinander verbunden sind.

10. Wiederverwendbare Schwimmwindel nach einem der vorhergehenden Ansprüche, **dadurch gekennzeichnet, dass** es sich bei der Innenschicht aus einer Maschenware oder einer Webware aus synthetischen Fasermaterialien um eine Polyester Innenschicht, vorzugsweise eine Polycabonat (PC), Polyethylenterephthalat (PET) und/oder Polyesterharz, handelt.

11. Wiederverwendbare Schwimmwindel nach einem der vorhergehenden Ansprüche, **dadurch gekennzeichnet, dass** die Außenschicht eine wasserdichte Beschichtung und/oder eine mikroporöse Membran aufweist.

12. Wiederverwendbare Schwimmwindel nach Anspruch 11, **dadurch gekennzeichnet, dass** es sich bei der Beschichtung um eine Kunststoff- und/oder Kunstharz Beschichtung, vorzugsweise um eine Polyurethan-Beschichtung, handelt.

13. Wiederverwendbare Schwimmwindel nach Anspruch 11, **dadurch gekennzeichnet, dass** es sich bei der mikroporösen Membran um eine synthetische und/oder halbsynthetische Polymermembran, vorzugsweise eine Polytetrafluoroethylen (PTFE) Membran, insbesondere eine gereckte Polytetrafluoroethylen (PTFE) Membran, handelt.

14. Wiederverwendbare Schwimmwindel nach einem der Ansprüche 11 bis 13, **dadurch gekennzeichnet, dass** die Beschichtung und/oder mikroporöse Membran im Wesentlichen ohne Nahtführung auf die Außenschicht aufgebracht ist.

15. Wiederverwendbare Schwimmwindel nach Anspruch 14, **dadurch gekennzeichnet, dass** die Beschichtung und/oder mikroporöse Membran mittels Welding (Druckschweißen), Verkleben und/oder Verschmelzen dauerhaft auf die Außenschicht aufgebracht ist.
